# EUROPEAN PATENT SPECIFICATION

(11) **EP 0 836 656 B1**
(45) Date of publication and mention of the grant of the patent: **10.12.2003**
(21) Application number: 96922568.9
(22) Date of filing: 26.06.1996
(51) Int. Cl.: D01F 8/06, D01F 8/10, D01F 8/14, D04H 1/54, D04H 1/42

(54) **WATER-DEGRADABLE MULTICOMPONENT FIBERS AND NONWOVENS**
WASSERABBAUBARE MEHRKOMPONENTENFASERN UND VLIESSTOFFE
FIBRES ET NON-TISSES COMPOSITES HYDRODEGRADABLES

(30) Priority: 30.06.1995 US 497667
(43) Date of publication of application: 22.04.1998
(73) Proprietor: Kimberly-Clark Worldwide, Inc., Neenah, WI 54956 (US)
(72) Inventor: JACKSON, David, Martin, Roswell, GA 30076 (US); POMPLUN, William, Seal, Neenah, WI 54956 (US)
(74) Representative: Grünecker, Kinkeldey, Stockmair & Schwanhäusser Anwaltssozietät
(86) International application number: US9610835
(87) International publication number: WO97002375

(56) References cited:
- EP-A- 0 569 145
- EP-A- 0 648 871
- WO-A-93/07199
- DATABASE WPI Section Ch, Week 9418 Derwent Publications Ltd., London, GB; Class A23, AN 94-148344 XP002017458 & JP,A,06 093 516 (KURARAY CO LTD) , 5 April 1994
- DATABASE WPI Section Ch, Week 9320 Derwent Publications Ltd., London, GB; Class A23, AN 93-162362 XP002017459 & JP,A,05 093 318 (UNITIKA LTD) , 16 April 1993
- DATABASE WPI Section Ch, Week 9434 Derwent Publications Ltd., London, GB; Class A96, AN 94-276136 XP002017460 & JP,A,06 207 324 (UNITIKA LTD) , 26 July 1994
- DATABASE WPI Section Ch, Week 9529 Derwent Publications Ltd., London, GB; Class A23, AN 95-220948 XP002017461 & JP,A,07 133 511 (TOYOBO KK) , 23 May 1995
- DATABASE WPI Section Ch, Week 9511 Derwent Publications Ltd., London, GB; Class A81, AN 95-078519 XP002017462 & JP,A,07 003 600 (DAIWABO CO LTD) , 6 January 1995
- PATENT ABSTRACTS OF JAPAN vol. 014, no. 017 (C-675), 16 January 1990 & JP,A,01 260017 (TORAY IND INC), 17 October 1989,

## Description

The present invention is directed to multicomponent fibers. More particularly, the present invention is directed to multicomponent fibers including bicomponent fibers which have at least one component which will permit bonding of the fibers to themselves and other types of fibers and wherein the same component is also degradable in an aqueous medium. Such fibers can be used to form fibrous nonwoven webs and can be used as components in such end products including, but not limited to, medical and health care related items, wipes and personal care absorbent articles such as diapers, training pants, incontinence garments, sanitary napkins, bandages and the like.

Solid waste disposal is becoming an ever increasing problem throughout the world. As landfills continue to fill up, there has been an increased demand for material source reduction in disposable products, the incorporation of more recyclable components in disposable products and the design of products which can be disposed of by means other than by incorporation into solid waste disposal facilities such as landfills.

One product area that has received particular attention with respect to solid waste disposal is disposable diapers. Most diapers in the United States are disposed of in landfills. Other proposed methods of disposal have included making all or a portion of the diapers flushable in public sewage systems and/or making their components more compatible with evolving composting and biomethanization techniques.

Diapers and almost all personal care products include in their design a bodyside cover, an absorbent core and some type of outercover to protect the clothing of the wearer from becoming soiled. Most bodyside covers and outercovers are made from fibrous nonwoven webs and/or films that are made from thermoplastic polymers such as polyolefins and polyesters. These portions of the product and especially diapers usually have to have a fairly high degree of integrity so that they remain intact during use. This same integrity, however, makes their disposal as, for example, through flushing in a toilet just that much more difficult.

In US patent no. 4,966,808 micro-fibers-generating conjugate fibers are disclosed wherein at least one conjugate component of said fibers has an island-in-sea structure and is exposed on the surface of said micro-fibers-generating conjugate fibers, and the sea part of said conjugate component is removable by a solvent treatment.

The problem underlying the present invention is to provide materials, components and product designs which make disposable items as personal care absorbent products more compatible with alternative disposal techniques such as toilet flushing, composting and biomethanization.

The problem is solved by a multicomponent fiber comprising a first component and at least a second component, said first component forming an exposed surface on at least a portion of said fiber, characterized in that the second component comprises a thermoplastic polymer and the first component comprises a thermally bondable and triggerably water-degradable polymer.

This first component may be made from a wide variety of water-degradable polymers including, for example, sulfonated polyester. The second component may be made from a thermoplastic fiber-forming polymer which is capable of being extruded through, for example, bicomponent fiber-forming equipment. Examples of such polymers include, polyolefins and polyesters. If desired, the second component also can be made from a polymer which is water-degradable though it generally will be more advantageous if the second polymer degrades at a rate which is slower than the degradation rate of the first component. Multicomponent fibers such as bicomponent fibers may be extruded in a number of forms including, but not limited to, concentric, and eccentric sheath/core configurations and side-by-side configurations. If desired, additional components also may be incorporated into the multicomponent fibers according to the present invention including other polymers and/or additives.

The multicomponent fibers according to the present invention may be used to form fibrous nonwoven webs using heat and/or pressure to bond the fibers together using the first component as the bonding agent. The webs may be formed completely from fibers according to the present invention or they may be blended with other fibers. Once the webs have been formed they may be used to form all or a portion of a number of end-use products including, but not limited to, personal care absorbent articles.

Figure 1 is a cross-sectional view of a concentric sheath/core bicomponent fiber according to the present invention.

Figure 2 is a cross-sectional view of an eccentric sheath/core bicomponent fiber according to the present invention.

Figure 3 is a cross-sectional view of a side-by-side bicomponent fiber according to the present invention.

The present invention is directed to a multicomponent fibers such as bicomponent fiber which includes a first component and a second component. For purposes of illustration only, the present invention will be described relative to a bicomponent fiber. It should be understood, however, that the scope of the invention is meant to encompass fibers with two or more components and thus "multicomponent" fibers. The first component provides at least two functions. First, it provides an exposed portion on the surface of the bicomponent fiber which will permit thermal bonding of the fiber to other fibers which may be the same as or different from the fibers of the present invention. Second, the first component of the bicomponent fiber of the present invention must be degradable when placed in an aqueous medium. As a result, the bicomponent fiber of the present invention can be used to form thermally bonded fibrous nonwoven webs. Then, when the web is exposed to an aqueous medium such as water in a toilet bowl, the fiber-to-fiber bond caused by the first component will degrade to a sufficient degree so that the fiber-to-fiber bonds will break apart thereby causing the fibrous nonwoven web to lose its integrity and break apart into smaller pieces or into individual fibers.

The second component is another fiber forming polymer which is less degradable in an aqueous medium when compared to the first component or totally non-degradable. Generally, the purpose of the second component will be to provide rigidity to the fiber and thus the resultant nonwoven web.

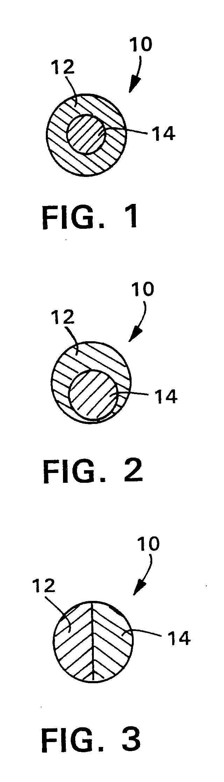

Referring to Figures 1 through 3, the bicomponent fibers 10 according to the present invention will have two or more components including a first component 12 and a second component 14 with the first component being "water-degradable". By this it is meant that when the first component 12 of the bicomponent fiber 10 is exposed to an aqueous liquid, including plain water such as is found in a toilet bowl or an aqueous mixture which may have added buffer, alkaline or acidic components or complex formers, the bond formed between the fibers by the first component will sufficiently weaken such that the fibers will begin to break apart by themselves or if necessary with agitation. To effect bonding, the first component 12 has a melting or softening temperature lower than the melting or softening temperature of the second component. In addition, as shown by Figures 1 through 3, the first component forms at least a portion of the exposed surface of the fiber 10, usually along the longitudinal axis of the fiber 10. Fiber cross-sections which provide such an exposed surface for the first component include, but are not limited to, a concentric sheath/core configuration such as is shown in Figure 1, an eccentric sheath/core configuration such as is shown in Figure 2 and a side-by-side configuration such as is shown in Figure 3. As a result, the first component serves as the bonding means for bonding the fibers together while the second component provides the structural rigidity to the fiber and the resultant nonwoven web.

The second component 14 is a polymer which has a higher melting or softening temperature than the first component 12 so that when thermal bonding is used to bond the bicomponent fibers together, the first component 12 is the primary means for effecting interfiber bonding. To accomplish this, it is generally desirable that the second component have a melting/softening temperature which is at least 10°C greater than the melting/softening temperature of the first component 12. Polymer or polymer blends which are relatively crystalline in nature will either have a specific melting temperature or a very narrow melting temperature range. Other polymers are more amorphous and thus will melt or soften over a broader temperature range. For polymers which are relatively crystalline, the melting temperature can be determined using differential scanning calorimetry (DSC) pursuant to ASTM Test Method E-794-85. For polymers which are more amorphous, the softening temperature or temperature range for the particular polymer, copolymer or blend can be determined using ASTM (Vicat) Test Method D-1525 (1993). Thus when choosing polymers for making the first and second components, the highest end of the melting or softening temperature range of the first component 12 should be at least 10°C lower than the lowest end of the melting or softening temperature range for the second component 14. As a result, when bonding of the bicomponent fibers is undertaken, the majority of the interfiber bonds will be via the first component and not the second component, provided the bonding temperature/conditions do not fall into the melting temperature range of the second component. Examples of polymers which are commonly used as the core or second component include, but are not limited to, polyesters and polyolefins such as polyethylene and polypropylene. Another factor in selecting the second component 14, is that it be sufficiently compatible with the first component from an adhesion standpoint so that the two components do not unduly separate from one another once the fiber 10 has been formed. This is especially true with respect to a side-by-side fiber configuration where there is little or no encapsulation of one component by the other as is the case with eccentric and concentric sheath/core fiber configurations.

Typically the material chosen to form the first component 12 will be more expensive than the second component 14. As a result, it is generally desirable to use as little of the first component when forming the fiber as is possible to reduce costs. To further reduce costs, it may be desirable to use other, lower cost fibers within the fibrous nonwoven web incorporating bicomponent fibers according to the present invention. Consequently, when mixing fibers, the fibers should be selected such that the first component of the bicomponent fiber according to the present invention will have the lowest melting/softening point of all of the fiber polymers being used in the nonwoven web.

As mentioned previously, the first component must be a material which is thermally bondable so as to be able to form fibrous nonwoven webs. The first component must also be water-degradable as outlined previously. Many polymers are degradable in essentially plain water such as tap water which typically has a pH in the range of about 6.5 to about 8.5. Thus, if fibrous nonwoven webs are made using water-degradable bicomponent fibers according to the present invention, then they will most likely be suitable for uses where they are exposed to very little or no water. Then, after use, they can be placed in water so that the sheaths can degrade and the fibrous nonwoven web can break apart. In other applications, fibrous nonwoven webs using water-degradable bicomponent fibers according to the present invention may be exposed to sufficient quantities of aqueous liquids during use, such that the first component would begin to degrade and break apart prematurely. Personal care absorbent articles such as diapers, incontinence garments, training pants and wet wipes are examples of such products which may be subject to this problem. To solve or reduce this problem, triggerably water-degradable polymers can be selected for the first component which are sensitive to or become degradable as a result of pH change, dissolved ion concentration change and/or temperature change in the aqueous environment.

As described in further detail below, certain first component polymers are water-degradable only when exposed to sufficient quantities of water within a certain pH range. Outside this range, they wilr not degrade. Thus it is possible to choose a pH-sensitive first component polymer which will be non-degradable in an aqueous liquid or liquids in one pH range, for example a pH of 3 to 5, but which become degradable in the pH range of normal tap water. See for example U.S. Patent No. 5,102,668 to Eichel et al.

Another mechanism which can be used to trigger water-degradability is ion sensitivity. Certain polymers contain acid-based (R-COO⁻) components which are held together by hydrogen bonding. In a dry state, these polymers remain solid. In an aqueous solution which has a relatively high cation concentration, such as urine, the polymer still will remain relatively intact. However, when the same polymer is later exposed to larger quantities of water with reduced ion content, such as can be found in a toilet bowl, the cation concentration will decrease and the hydrogen bonding will begin to break apart. As this happens, the polymer, itself, will begin to break apart in the water. See for example, U.S. Patent No. 4,419,403 to Varona which is incorporated herein by reference in its entirety.

Yet another means for rendering a polymer degradable in water is through the use of temperature change. Certain polymers exhibit a cloud point. As a result, these polymers will precipitate out of solution at a particular temperature - the cloud point. These polymers can be used to form fibers which are insoluble in water above a certain temperature but which become soluble and thus degradable in water at a lower temperature. As a result, it is possible to select or blend a polymer which will not degrade in body fluids, such as urine, at or near body temperature (37°C) but which will degrade when placed in water at temperatures at or below room temperature (23°C). An example of such a polymer is polyvinylmethylether which has a cloud point of 34°C. When this polymer is exposed to body fluids such as urine at 37°C, it will not degrade as this temperature is above its cloud point (34°C). However, if the polymer is placed in water at room temperature (23°C), the polymer will go back into solution as it is now exposed to water at a temperature below its cloud point. Consequently, the polymer will begin to degrade.

First component polymers can be categorized according to the means by which they are found to be stable in specific fluid environments. These environmental conditions are: low volume water or body fluids, regulated pH conditions (such as found in wet-wipe storage solutions or buffered systems), high cationic strength solutions (for example, baby or adult urine and menses), and variable temperature conditions (for example, body temperature versus room temperature or colder tap water).

Referring to Table I, examples of first component polymers that are stable in low water volume solution environments (for example, panti-liners, light incontinence products and baby or adult wipes), could be NP2068, NP2074 or NP2120 polyamides as supplied by the H.B. Fuller Company of Vadnais Heights, Minnesota. The Fuller materials are aliphatic polyamides which are completely cold water soluble and process in fiber spinning very similarly to low density polyethylene. Other polymers capable of degrading in aqueous mixtures or toilet water are poly (vinyl alcohol) graft copolymers supplied by the Nippon Synthetic Chemical Co., Ltd., Osaka, Japan, coded Ecomaty AX2000, AX10000 and AX-300G. The Nippon polymers are cold water soluble but somewhat slower in their rate of solubility than the Fuller polymers. Yet another first component polymer could be a polyether blockamide, coded Pebax MX1074, supplied by Atochem (USA) located in Philadelphia, Pennsylvania. The Pebax MX1074 polymer is composed of epsilon-caprolactam (Nylon 12) and tetramethylene glycol monomers. These monomers are polymerized to make a series of polyether block amide copolymers. The Pebax polymer is not water soluble but is water-swellable, and therefore could also be used in a higher water volume environment as well. The Fuller polymers can be matched to a second component (core) polymer with a softening or melting temperature at least about 10°C higher, such as would be the case with polypropylene. The Nippon or Atochem polymers can be matched with a higher melting temperature range second component polymer such as polypropylene or poly (butylene terephthalate).

**TABLE I**

| SHEATH POLYMERS FOR BICOMPONENT FIBERS | | | |
|---|---|---|---|
| Polymer Type | Melt Flow* Or Viscosity | DSC Soft Temp (Range) | Matched Core Polymer |
| H.B. Fuller Code NP-2120 | 410 Pa.s @204°C | 142°C-158°C | Polypropylene |
| H.B. Fuller Code NP-2068 | 95 Pa.s @204°C | 128°C-145°C | Polypropylene |
| H.B. Fuller Code NP-2074 | 290 Pa.s @204°C | 133°C-145°C | Polypropylene |
| ATOCHEM PEBAX MX1074 | MFR = 1-3 | 158°C | Polybutylterephthalate |
| Nippon-Gohsei ECOMATY AX10000 | MFR = 100 | 180°C | Polybutylterephthalate |
| Findley Blend | 200 Pa.s | 117°C | Polyethylene |
| N-10, | @140°C | | |
| Acrylate ester/ acrylic or methacrylic acid | | | |
| Findley Blend | 370 Pa.s | 131°C | Polypropylene |
| H-10, | @160°C | | |
| Acrylate ester/ acrylic or methacrylic acid | | | |
| Findley Blend | 30 Pa.s | | |
| X-10, | @190°C | ― | ― |
| Acrylate ester/ acrylic or methacrylic acid | | | |
| Eastman | 300 Pa.s | 120°C-130°C | Polyethylene |
| Code AQ38S | @200°C | | |

| | | | |
|---|---|---|---|
| *ASTMD Test Method D-1238-906 (2.16 kg load at 190°C for polyethylene) | | | |

First component polymers that are stable in a specific pH range, as for example, from pH 3-5 could be acrylate ester/acrylic or methacrylic acid copolymers and blends coded N-10, H-10 or X-10 as supplied by Findley Adhesives, Inc. of Milwaukee, Wisconsin. The Findley materials are stable at body pH conditions (or when buffered against body fluids), but will break-up rapidly in toilet water during the flushing process (excess water) thus increasing the pH towards neutral. The Findley polymers can be matched with a higher melting temperature range second component polymer such as polyethylene or polypropylene.

First component polymers that are stable in high cation concentration solution environments (for example, baby or adult urine and menses) could be sulfonated polyesters coded AQ29, AQ38, or AQ55, as supplied by the Eastman Chemical Company of Kingsport, Tennessee. The Eastman AQ38 polymer is composed of 89 mole percent isophthalic acid, 11 mole percent sodium sulfoisophthalic acid, 78 mole percent diethylene glycol and 22 mole percent 1,4-cyclohexanedimethanol. It has a nominal molecular weight of 14,000 Daltons, an acid number less than two, a hydroxyl number less than 10 and a glass transition temperature of 38°C. other examples could be blends of poly(vinyl alcohol) or copolymers of poly(vinyl alcohol) blended with polyacrylic or methacrylic acid, or polyvinylmethyl ether blended with polyacrylic or methacrylic acid. The Eastman polymers are stable in high cationic solution environments, but will break-up rapidly in toilet water during the flushing process (excess water) thus reducing cation concentration. The Eastman polymers can be matched with a higher melting temperature range second component polymer such as polyethylene.

First component polymers that are stable at body temperature conditions (that is a temperature of 37°C) could be any polymer that exhibits a "cloud point" at or near body temperature. The cloud point, or inverse solubility temperature is a useful property for "triggering" a change in physical state, such as water solubility. For example, polyvinylmethylether has a cloud point temperature of 34°C above which it is no longer water soluble, but at room temperature (near 23°C) it is completely water degradable. Blends of polyvinylmethylether may be considered as well. Polyvinylmethylether can be matched with higher melting temperature range second component polymers such as polyethylene.

The methods for making bicomponent fibers are well known and need not be described herein in detail. To form a bicomponent fiber, generally, two polymers are extruded separately and fed to a polymer distribution system where the polymers are introduced into a segmented spinneret plate. The polymers follow separate paths to the fiber spinneret and are combined in a spinneret hole which comprises either two concentric circular holes thus providing a sheath/core type fiber or a circular spinneret hole divided along a diameter into two parts to provide a side-by-side type fiber. The' combined polymer filament is then cooled, solidified and drawn, generally by a mechanical rolls system, to an intermediate filament diameter and collected. Subsequently, the filament is "cold drawn", at a temperature below its softening temperature, to the desired finished fiber diameter and is crimped/texturized and cut into a desirable fiber length. Bicomponent fibers can be cut into relatively short lengths, such as staple fibers which generally have lengths in the range of 25 to 51 millimeters (mm) and short-cut fibers which are even shorter and generally have lengths less than 18 millimeters. See, for example, U.S. Patent No. 4,789,592 to Taniguchi et al. and U.S. Patent No. 5,336,552 to Strack et al,.

Fibrous nonwoven webs may be made completely from the fibers of the present invention or they may be blended with other fibers. The length of the fibers will depend upon the particular end use. Where the fibers are to be degraded in water, for example, in a toilet, it is advantageous if the fiber lengths are maintained at or below about 15 millimeters (mm).

The water-degradable bicomponent fibers of the present invention may be used to form fibrous nonwoven webs for a number of uses.

Personal care absorbent articles include such items as diapers, training pants, feminine hygiene products such as sanitary napkins, panti-liners and tampons, incontinence garments and devices, bandages. The most basic design of all such articles typically includes a bodyside liner, an outercover and an absorbent core disposed between the bodyside liner and the outercover. Generally, the bodyside liner and the outercover are sealed about their peripheries so as to encapsulate the absorbent core and thus make it possible to entrap and retain any fluids contained within the absorbent core. Depending upon the design of the particular personal care absorbent article, other components also may be included. Thus, the product may include such things as elastic side panels, fluid containment flaps, fastening devices and other layers of fluid transfer or retention materials. Where suitable, fibrous nonwoven webs incorporating such water-degradable bicomponent fibers may be used to form all or a portion of the foregoing components.

Other potential uses for the fibers and nonwoven webs according to the present invention include, wet and dry wipers, articles of clothing and any other nonwovens or composites where a water-degradable feature might be advantageous.

Having thus described the various parameters of the present invention, several samples of the water-degradable bicomponent fibers and nonwoven webs were made.

### EXAMPLE I

In Example I a sheath/core water-degradable bicomponent fiber was made using a high density polyethylene core and a sulfonated polyester sheath in a 50/50 weight ratio. The core polymer was NCPE 1961 high density polyethylene from Neste Oy, of Espoo, Finland with a melting temperature range of 140-150°C. The sheath polymer was AQ38S sulfonated polyester from the Eastman Chemical Company of Kingsport, Tennessee with a melting temperature range of 120-130°C. The fibers were produced utilizing a bicomponent fiber line, at an initial fiber diameter of 6 decitex (dtex) and were then cold drawn at a draw temperature of between 50 and 60°C to a final diameter of 4 dtex. The fibers were mechanically crimped in a stuffer box and cut to a length of 6 mm. A neat iso-propyl myristate spin finish was also used.

Once the fibers had been formed, they were then air laid in a 50/50 weight ratio, based upon the total weight of the web, the fibrous nonwoven web having a basis weight of 25 to 30 grams per square meter (gsm) in combination with one of three other 1.7 dtex by 6 mm fibers. The three other fibers included a polyester fiber from the Dupont Fiber Company of Wilmington, Delaware, a polyester fiber from EMS Grilon of Domat/Ems, Switzerland and a rayon fiber from Courtaulds, Ltd. of Coventry, England. All three webs were bonded using a two step bonding process. In the first step for the blended rayon/water-degradable bicomponent fiber web, the fibers were through-air bonded at a temperature of 132°C followed by point bonding in a pair of patterned bonding rolls with a bond area of about 15 percent at a temperature of 90°C. The blended polyester/water-degradable fiber webs were bonded at the same temperatures but bonding of the web containing the Dupont polyester fibers could not be perfected due to excessive shrinking of the polyester fibers. The fibers of the other two webs that did bond, formed fiber-to-fiber bonds via the sulfonated polyester sheath component. When the webs were placed in room temperature water and agitated, the fiber bonds degraded and the webs broke apart.

### EXAMPLE II (Reference)

In Example II, a second sheath/core water-degradable fiber was formed using a core made from Vestodur 1000 poly(butylene terephthalate) polymer from Hüls, GmbH a subsidiary of Veba AG of Duesseldorf, Germany. The PBT polymer had a melting temperature of 250°C. The water-degradable sheath was made from AX2000 poly(vinyl alcohol) from Nippon Synthetic Chemical Company, Ltd. of Osaka, Japan. It had a melting temperature of 225°C. As with the fibers in Example I, the sheath/core polymer weight ratio was 50/50. The fibers were initially drawn to 14 dtex and then cold drawn to 5 dtex at a temperature of 130°C. Following formation, the fibers were mechanically crimped and cut to a length of 6 mm. The same iso-propyl myristate spin finish was used on the fibers.

The water-degradable sheath/core bicomponent fibers were again blended with the same Ems Grilon polyester fibers and Courtaulds rayon fibers used in Example I in a 50/50 weight percent blend of polyester and bicomponent fibers and rayon/bicomponent fibers. The two air laid webs had basis weights of between 25 and 30 gsm. Bonding of the two webs was unsuccessful due to equipment limitations. Despite this, the poly(vinyl alcohol) sheaths of the bicomponent fibers did degrade and became slimy when placed in water.

## Claims

1. A multicomponent fiber comprising a first component and at least a second component, said first component forming an exposed surface on at least a portion of said fiber, **characterized in that** the second component comprises a thermoplastic polymer and the first component comprises a thermally bondable and triggerably water-degradable polymer.

2. The multicomponent fiber of claim 1, wherein said fiber has a sheath/core configuration, said first component forming said sheath.

3. The multicomponent fiber of claim 1, wherein said fiber has a side-by-side configuration.

4. The multicomponent fiber of claim 1, wherein said second component is a thermoplastic fiber-forming polymer.

5. The multicomponent fiber of claim 4, wherein said thermoplastic fiber-forming polymer is a polyolefin.

6. The multicomponent fiber of claim 4, wherein said thermoplastic fiber-forming polymer is a polyester.

7. A nonwoven web comprising a plurality of fibers, at least a portion of said plurality of fibers comprising the multicomponent fibers of any of claims 1 to 6.

8. A personal care absorbent article which includes the nonwoven web according to claim 7.

9. A wiper including the nonwoven web according to claim 7.

10. A diaper including the nonwoven web according to claim 7.

11. A training pant including the nonwoven web according to claim 7.

12. An incontinence garment including the nonwoven web according to claim 7.

13. A sanitary napkin including the nonwoven web according to claim 7.

14. A panti-liner including the nonwoven web according to claim 7.

15. A bandage including the nonwoven web according to claim 7.

## Patentansprüche

1. Mehrkomponenten-Faser, die eine erste Komponente und mindestens eine zweite Komponente umfasst, wobei die erste Komponente auf mindestens einem Teil der Faser eine freiliegende Oberfläche bildet,
**dadurch gekennzeichnet, dass** die zweite Komponente ein thermoplastisches Polymer umfasst und die erste Komponente ein thermisch bindungsfähiges und triggerbares, in Wasser abbaubares Polymer umfasst.

2. Mehrkomponenten-Faser nach Anspruch 1, die eine Hüllen-Kern-Konfiguration hat, wobei die erste Komponente die Hülle bildet.

3. Mehrkomponenten-Faser nach Anspruch 1, die eine Seite-an-Seite-Konfiguration hat.

4. Mehrkomponenten-Faser nach Anspruch 1, in der die zweite Komponente ein thermoplastisches, faserbildendes Polymer ist.

5. Mehrkomponenten-Faser nach Anspruch 4, in der das thermoplastische faserbildende Polymer ein Polyolefin ist.

6. Mehrkomponenten-Faser nach Anspruch 4, in der das thermoplastische faserbildende Polymer ein Polyester ist.

7. Vliesstoffbahn, die eine Vielzahl von Fasern umfasst, wobei mindestens ein Teil der Vielzahl von Fasern die Mehrkomponenten-Fasern nach einem der Ansprüche 1 bis 6 umfasst.

8. Absorptionsfähiger Körperpflege-Artikel, der die Vliesstoffbahn nach Anspruch 7 enthält.

9. Wischtuch, das die Vliesstoffbahn nach Anspruch 7 enthält.

10. Windel, welche die Vliesstoffbahn nach Anspruch 7 enthält.

11. Trainingshose, welche die Vliesstoffbahn nach Anspruch 7 enthält.

12. Inkontinenz-Kleidungsstück, das die Vliesstoffbahn nach Anspruch 7 enthält.

13. Damenbinde, welche die Vliesstoffbahn nach Anspruch 7 enthält.

14. Schlüpfereinlage, welche die Vliesstoffbahn nach Anspruch 7 enthält.

15. Bandage, welche die Vliesstoffbahn nach Anspruch 7 enthält.

## Revendications

1. Fibre multicomposée comprenant un premier composant et au moins un second composant, ledit premier composant formant une surface à découvert sur au moins une portion de ladite fibre, **caractérisée en ce que** le second composant comprend un polymère thermoplastique et le premier composant comprend un polymère liable thermiquement et dont la dégradation par l'eau est provoquée par un déclencheur.

2. Fibre multicomposée selon la revendication 1, dans laquelle ladite fibre a une configuration écorce-noyau, ledit premier composant formant ladite écorce.

3. Fibre multicomposée selon la revendication 1, dans laquelle ladite fibre a une configuration côte à côte.

4. Fibre multicomposée selon la revendication 1, dans laquelle ledit second composant est un polymère thermoplastique formateur de fibres.

5. Fibre multicomposée selon la revendication 4, dans laquelle ledit polymère thermoplastique formateur de fibres est une polyoléfine.

6. Fibre multicomposée selon la revendication 4, dans laquelle ledit polymère thermoplastique formateur de fibres est un polyester.

7. Voile non-tissé comprenant une pluralité de fibres, au moins une portion de ladite pluralité de fibres comprenant des fibres multicomposées selon l'une quelconque des revendications 1 à 6.

8. Article absorbant d'hygiène intime qui comprend le voile non-tissé selon la revendication 7.

9. Lingette comprenant le voile non-tissé selon la revendication 7.

10. Change pour nourrissons comprenant le voile non-tissé selon la revendication 7.

11. Culotte d'apprentissage de la propreté comprenant le voile non-tissé selon la revendication 7.

12. Vêtement pour l'incontinence adulte comprenant le voile non-tissé selon la revendication 7.

13. Serviette hygiénique comprenant le voile non-tissé selon la revendication 7.

14. Protège-slip comprenant le voile non-tissé selon la revendication 7.

15. Pansement comprenant le voile non-tissé selon la revendication 7.
